# FASCICULE DE BREVET EUROPEEN

(11) **EP 0 474 847 B1**
(45) Date de publication et mention de la délivrance du brevet: **13.09.1995**
(21) Numéro de dépôt: 91907500.2
(22) Date de dépôt: 28.03.1991
(51) Int. Cl.: C12M 3/00, C12M 1/12

(54) **DISPOSITIF DE CULTURE CELLULAIRE**
ZELLKULTURANLAGE
CELL CULTURE DEVICE

(30) Priorité: 30.03.1990 FR 9004092
(43) Date de publication de la demande: 18.03.1992
(73) Titulaire: BERTIN & CIE, F-78373 Plaisir Cédex (FR)
(72) Inventeur: BINOT, Patrick, Rentilly, F-77600 Bussy-S.-Georges (FR); COGNARD, Dominique, F-78280 Guyancourt (FR); DUFAU, Frédéric, F-78170 La Celle-S.-Cloud (FR); HACHE, Jean, F-78190 Trappes (FR)
(74) Mandataire: Orès, Bernard
(86) Numéro de dépôt international: FR9100246
(87) Numéro de publication internationale: WO9115570

(56) Documents cités:
- EP-A- 113 328
- WO-A-86/06094
- WO-A-90/13639
- US-A- 4 610 789
- BIOTECHNOLOGY & BIOENGINEERING, vol. 28, no. 3, March 1986, John Wiley & Sons Inc., New York, NY (US); J.P. THARAKAN et al., pp. 329-342/

## Description

La présente invention est relative à un dispositif de culture cellulaire ou bioréacteur en masse et en continu permettant notamment l'obtention améliorée des produits desdites cultures.

On connaît actuellement plusieurs méthodes et dispositifs de culture cellulaire.

Les cellules peuvent être mises en culture sur une surface lisse telle que verre ou plastique approprié (boîtes ou tubes). De telles cultures ne permettent pas de réaliser une croissance cellulaire importante et nécessitent de grandes quantités de nutriments.

En particulier, les Brevets US 3 883 393 (USA), 3 997 396 (MONSANTO Co.), 4 087 327, 4 201 845 (MONSANTO Co.), 4 220 725 (USA) et 4 391 912 décrivent des systèmes dans lesquels les cellules sont cultivées sur ou à proximité de fibres creuses.

Le Brevet 4 201 845 (MONSANTO Co.) décrit notamment un réacteur de culture cellulaire comprenant une chambre de culture dans laquelle une couche de fibres creuses pour l'alimentation gazeuse est prise en sandwich entre une membrane microporeuse pour la distribution du milieu de culture et une membrane microporeuse servant de barrière à la diffusion des cellules à l'extérieur de ladite chambre. Le flux de milieu est dirigé vers le haut et transversalement au plan des fibres.

Ce dispositif présente cependant un certain nombre d'inconvénients et notamment des risques de colmatage de la membrane distributrice de milieu de culture, en raison de l'attaque frontale de ladite membrane, lequel colmatage entraîne des inégalités locales de débit et une non homogénéité de la distribution des nutriments.

D'autres dispositifs de cultures cellulaires ont également été décrits :
. la Demande Internationale WO 90/13639 INVITRON CORPORATION décrit un appareil qui comprend une enceinte contenant une chambre de culture cellulaire délimitée par une cartouche d'entrée d'un milieu convenable et une cartouche d'élimination de produits ;
. la Demande Internationale WO 86/06094 décrit un ensemble composite de membranes de filtration pour effectuer simultanément ou pratiquement simultanément une réaction biologique et la séparation des produits obtenus ;
. la Demande de Brevet EP 0 113 328 décrit un dispositif de culture de cellules *in vitro* dans lequel les cellules sont retenues dans une matrice rigide alimentée en milieu nutritionnel, à travers un tube de porosité relativement faible tandis que les produits cellulaires et le milieu nutritif épuisé sont extraits à travers un tube de porosité relativement élevée, une alimentation en oxygène étant prévue, grâce à une membrane tubulaire sélectivement perméable.

Dans certains de ces dispositifs, et notamment dans le dispositif MONSANTO, la distribution et la répartition du milieu vers les cellules n'est pas réalisée de façon homogène sur toute la surface de la plaque, pouvant entraîner des disparités difficiles à maîtriser dans le métabolisme des cellules, suivant leur position dans le dispositif et des difficultés d'extrapolation de taille du dispositif, notamment dans le cadre d'une réalisation industrielle.

La Demanderesse s'est en conséquence donné pour but de pourvoir à un bioréacteur ou dispositif de culture cellulaire qui répond mieux aux besoins de la pratique que les dispositifs de l'Art antérieur, notamment en ce qu'il permet la production de produits de culture, en continu et en masse, à partir de cultures maintenues à haute concentration cellulaire supérieure à 5.10⁸/cm³, sur une longue période sans entraîner de colmatage c'est-à-dire sans diminution du débit de nutriments dans tout l'espace cellulaire et l'utilisation d'un espace cellulaire de dimension importante, mieux adapté à l'échelle industrielle.

La présente invention a pour objet un dispositif de culture cellulaire ou bioréacteur pour la production de métabolites, du type comprenant un espace de culture cellulaire dans lequel les cellules sont confinées dans un milieu liquide, ledit espace étant délimité par des parois dont au moins trois sont sélectivement perméables, la première au milieu nutritionnel frais, la seconde aux fluides gazeux et la dernière au milieu nutritionnel usé et des moyens de circulation des fluides dans ledit dispositif, lequel dispositif est caractérisé en ce que :
- la première paroi est constituée par une nappe de tubes à paroi perméable au milieu nutritionnel frais, rigides et de diamètre d'environ un centimètre, montés en parallèle entre un collecteur d'entrée et un collecteur de sortie dans un circuit en boucle fermée, incluant des moyens d'apport dudit milieu nutritionnel frais et des moyens de mise sous pression, lesquels tubes sont parcourus chacun de bout en bout par un débit de milieu frais plusieurs fois supérieur à celui traversant sa paroi, pour maintenir une pression entraînant une perte de charge, lorsque le milieu frais traverse les parois des tubes, supérieure à 200 mbars,
- la seconde paroi est constituée par un ensemble de capillaires à paroi perméable aux gaz, disposés à intervalle sensiblement égaux pour former, de l'un et/ou de l'autre côté de la nappe de tubes, un/plusieurs sous-ensembles à distribution homogène pour les échanges gazeux avec les cellules,
- la troisième paroi comprend au moins un tamis micronique constituant chaque paroi aval de l'espace de culture cellulaire, pour assurer le confinement des cellules et permettant la sortie du milieu usé.

On entend, au sens de la présente invention, par milieu frais, un milieu riche en nutriments, fourni aux cellules, pour leurs besoins et leur croissance.

On entend par milieu usé, un milieu appauvri en nutriments et contenant les métabolites excrétés par lesdites cellules.

Selon une caractéristique avantageuse de ce dispositif, le débit du milieu parcourant chaque tube correspond à une vitesse d'écoulement, d'environ 1 dm/s, qui garantit la filtration tangentielle du milieu frais à travers la paroi desdits tubes.

Lesdits tubes ont une paroi ayant des pores d'un diamètre permettant le passage des molécules de nutriments et notamment le passage des molécules d'un poids moléculaire supérieur à 150 kDa.

De tels tubes ont l'avantage d'être stérilisables et permettent d'obtenir une homogénéité de distribution des nutriments, avec une dispersion maximale de l'ordre de 10 %.

Le circuit en boucle est maintenu sous une pression appropriée, calculée de telle sorte, qu'elle permette d'obtenir le rapport recherché entre le débit circulant et le débit traversant et de vaincre la pression capillaire.

Lesdits sous-ensembles de capillaires peuvent être simples, c'est-à-dire sous la forme d'une seule couche ou bien être eux-mêmes formés de plusieurs couches superposées de capillaires parallèles entre eux, les capillaires d'une couche régnant au droit des intervalles entre capillaires des deux couches adjacentes.

Les cellules qui peuvent être mises en culture dans le dispositif de la présente invention sont notamment les microorganismes et les cellules animales ou végétales procaryotes et eucaryotes. Elles peuvent éventuellement avoir été modifiées ; elles peuvent éventuellement être fixées sur un support.

Lesdits capillaires diffusent le gaz qui les traversent (air, O₂, N₂, CO, CO₂ etc...) de manière homogène à l'intérieur de l'espace de culture cellulaire.

Selon une disposition préférée de cette caractéristique, lesdits capillaires sont des fibres creuses de diamètre d'environ 1 millimètre, à paroi hydrophobe.

La multiplication de la surface de distribution des gaz et notamment de l'oxygène entraîne une diminution des distances de diffusion et permet ainsi, grâce à une oxygénation plus intense et plus homogène sans stress mécanique, une viabilité nettement améliorée des cellules ainsi qu'une évacuation du CO₂ produit par les cellules.

De manière inattendue, le mode d'alimentation par filtration tangentielle au travers de tubes poreux évite les problèmes de colmatage, assure une distribution homogène du milieu dans l'espace de culture, entraînant ainsi une meilleure productivité et homogénéité de l'ensemble des cellules.

De plus, l'apport de nutriments à l'aide de tubes permet de simplifier et de maîtriser l'hydrodynamique globale du bioréacteur et la mise en place d'un réseau de fibres d'aération permet d'apporter directement l'oxygène au milieu des cellules dans un espace cellulaire important et ainsi de commander, de façon indépendante l'apport de nutriments, l'oxygénation et l'extraction des produits de la culture cellulaire.

Selon une caractéristique avantageuse de ce dispositif, ledit tamis micronique recouvre au moins chaque face libre aval de l'ensemble des capillaires.

Selon une disposition préférée de cette caractéristique, ladite troisième paroi est une membrane poreuse comprenant des pores d'un diamètre compris entre 1 et 10 µm et avantageusement sélectionnée parmi les membranes organiques et les membranes métalliques.

Selon une autre caractéristique avantageuse d'un dispositif conforme à l'invention, les capillaires sont orientés parallèlement au plan de la nappe des tubes.

Selon une disposition préférée de cette caractéristique, les capillaires sont parallèles aux tubes.

Selon une modalité préférée de cette disposition, lesdits capillaires sont aussi répartis dans l'espace cellulaire libre entre les tubes.

Selon une autre caractéristique avantageuse d'un dispositif conforme à l'invention, la nappe de tubes et chaque ensemble ou sous-ensemble de capillaires sont séparés par un tamis micronique semblable au tamis micronique formant la troisième paroi dudit dispositif.

Selon une autre caractéristique avantageuse d'un dispositif conforme à l'invention, il constitue un module unilatéral, comprenant un seul ensemble de capillaires situé d'un côté de la nappe de tubes et au moins un tamis micronique aval ou de sortie.

Selon encore une autre caractéristique avantageuse d'un dispositif conforme à l'invention, il constitue un module bilatéral ou symétrique, comprenant au moins deux sous-ensembles de capillaires s'étendant respectivement de chaque côté de la nappe de tubes et au moins un tamis micronique aval ou de sortie, recouvrant chaque sous-ensemble de capillaires.

Selon encore une autre caractéristique avantageuse d'un dispositif conforme à l'invention, il comprend au moins deux modules symétriques superposés, les espaces de sortie et tamis microniques intermédiaires étant éventuellement supprimés.

Ledit dispositif peut également comprendre une enceinte traversée par au moins deux jeux de nappes de tubes, l'espace entre les tubes et les tamis microniques d'extrémité étant empli de capillaires parallèles auxdits tubes.

Le bioréacteur conforme à l'invention permet de résoudre un certain nombre de problèmes :
- cellules :: - augmentation de productivité et bonne utilisation du volume de l'espace cellulaire ;
- diminution des risques d'endommagement ;
- augmentation de la viabilité et concentration cellulaire augmentée et plus homogène, par diminution de la distance de diffusion de l'oxygène et par un apport homogène de nutriments ;
- possibilité de cultiver des cellules fixées ou des cellules libres.
- nutriments :: - diminution des besoins en sérum, du fait de la forte concentration cellulaire, ce qui entraîne une diminution du coût ;
- boucle de recirculation des nutriments ;
- limitation des risques de colmatage à l'alimentation, par l'application d'une filtration tangentielle.
- production :: le dispositif conforme à l'invention a l'avantage d'être particulièrement bien adapté aux applications industrielles, nécessitant des facilités d'extrapolation en taille.

Outre les dispositions qui précèdent, l'invention comprend encore d'autres dispositions, qui ressortiront de la description qui va suivre, qui se réfère aux dessins annexés dans lesquels :
- la figure 1 représente une vue schématique d'ensemble d'un bioréacteur conforme à l'invention ;
- la figure 2 représente une vue schématique du bioréacteur en coupe orthogonale par rapport aux tubes ;
- la figure 3 représente une vue en coupe du bioréacteur selon la ligne 3-3 de la figure 2 ;
- la figure 4 est une vue en coupe de la zone d'alimentation selon la ligne 4-4 de la figure 2 ;
- les figures 5a à 5f représentent schématiquement des modes de réalisation différents du bioréacteur conforme à l'invention.

Il doit être bien entendu, toutefois, que ces dessins et les parties descriptives correspondantes, sont donnés uniquement à titre d'illustration de l'objet de l'invention, dont ils ne constituent en aucune manière une limitation.

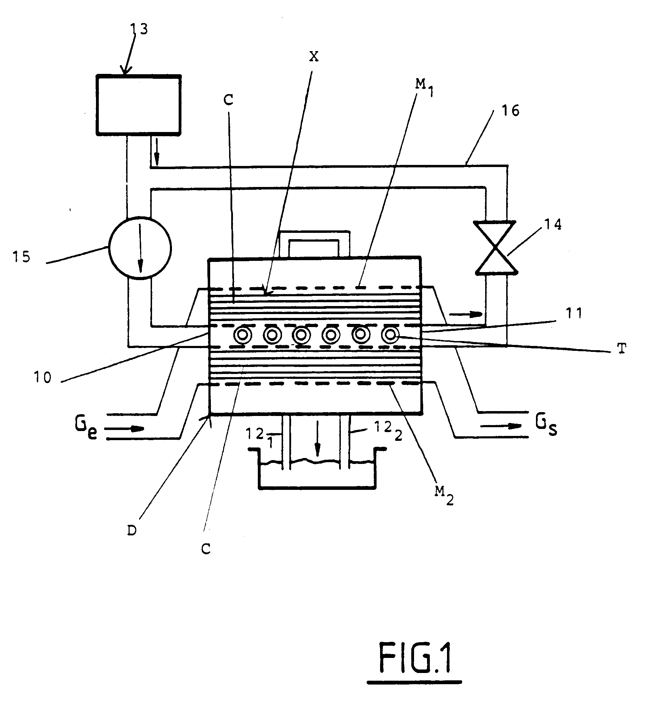

On se réfère d'abord aux figures 1 à 4, qui illustrent des modes de réalisation du bioréacteur conforme à l'invention.
La figure 1 représente une vue schématique d'un bioréacteur conforme à l'invention, comprenant dans un carter D délimité par un espace de culture cellulaire X :
   . un premier tamis micronique M₁ qui assure le confinement des cellules dans l'espace X mais permet le passage du milieu usé à récupérer,
   . un premier sous-ensemble de capillaires C qui assurent le passage des fluides gazeux et qui sont disposés entre le tamis M₁ et une nappe de tubes T, parallèles entre eux et qui assurent le passage du milieu nutritif frais vers les cellules,
   . un second tamis micronique M₂ qui assure le confinement des cellules dans l'espace X, à l'autre extrémité dudit espace et un deuxième sous-ensemble de capillaires C, disposé entre le tamis M₂ et la nappe de tubes T.

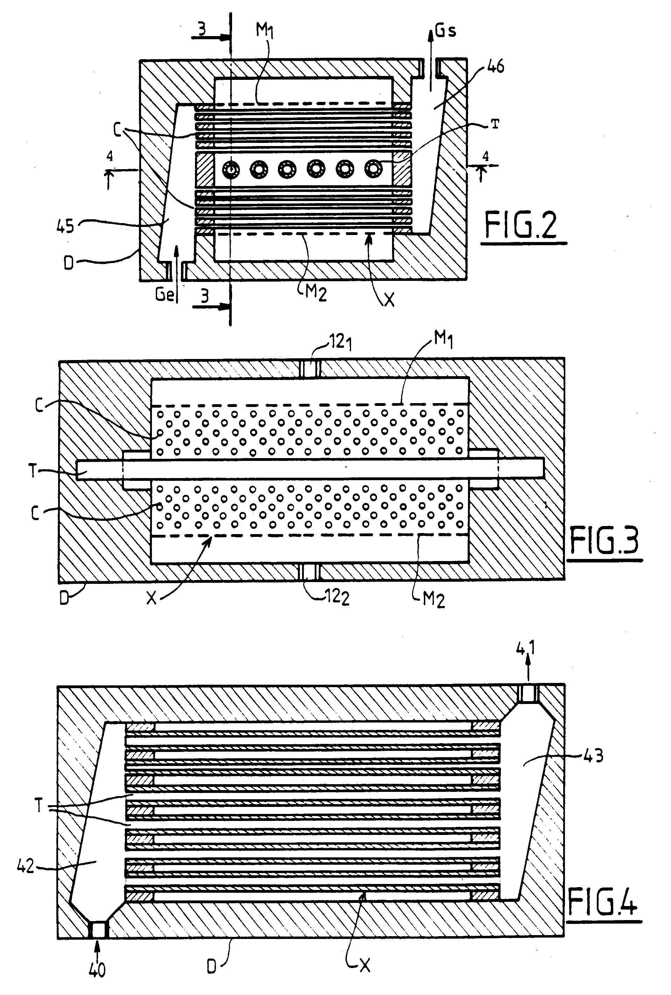

Les tubes T sont montés en parallèle entre deux collecteurs 42 et 43 (figure 4) alimentés suivant un circuit en boucle fermée incluant une tubulure d'entrée 10 (figure 1), 40 (figure 4) d'apport du milieu nutritionnel frais et une tubulure 11 (figure 1), 41 (figure 4) de sortie dudit milieu frais à recycler, lesquelles tubulures sont reliées entre elles par une canalisation 16 (figure 1).

Des conduits 12₁ et 12₂ de sortie du milieu usé (figures 1 et 3), débouchant en aval des tamis M₁ et M₂ permettent d'extraire ledit milieu usé collecté entre les tamis et le carter D.

Des moyens d'entrée Ge et de sortie Gs des fluides gazeux (figure 2) sont reliés respectivement à un volume d'alimentation 45 des capillaires C et un volume 46 d'échappement des capillaires.

Dans la réalisation illustrée à la figure 1 :
- les capillaires C sont, dans le même plan, perpendiculaires aux tubes T et sont disposés en deux sous-ensembles, de part et d'autre de la nappe de tubes T ;
- le dispositif comprend en outre, associé à la canalisation 16 de circulation de milieu frais, des moyens de mise sous pression 14, 15 dudit milieu frais. Le milieu frais sous pression circulant dans les tubes T, entre l'entrée 10 et la sortie 11, est recyclé en permanence dans une boucle alimentée à partir d'un réservoir 13 branché sur la canalisation 16 ; une filtration tangentielle dudit milieu frais à travers lesdits tubes T dans l'espace de culture cellulaire X peut ainsi être assurée.

Dans une réalisation avantageuse du dispositif de l'invention, le moyen 14 est réalisé par une vanne d'étranglement introduisant une perte de charge et le moyen 15 est ménagé par une pompe.

Dans une variante, le bioréacteur conforme à l'invention peut également comprendre les moyens suivants, non représentés à la figure 1 :
- en amont de l'entrée gazeuse Ge, on peut trouver un filtre stérile ;
- l'espace de culture cellulaire X peut être associé à un dispositif de régulation de la température ;
- la boucle de recirculation de milieu frais (tubulures 10 et 11) et les conduits d'évacuation de milieu usé (moyens 12₁ et 12₂) peuvent être associés à des réservoirs appropriés.

Le fonctionnement de tels bioréacteurs est le suivant :
Après stérilisation du carter D et réglage de la température, du pH, de la concentration en oxygène, du débit en milieu approprié additionné en sérum, on inocule une quantité appropriée de cellules dans l'espace de culture cellulaire X et l'on introduit le milieu frais par le moyen d'apport 40 vers le collecteur 42 (figure 4). On surveille quotidiennement la concentration en nutriments du milieu frais ainsi que les paramètres précisés ci-dessus (température, pH, concentration en O₂). Le milieu frais s'écoule à travers les tubes T à une vitesse qui garantit la filtration tangentielle du milieu frais à travers les parois desdits tubes et assure ainsi une homogénéité de l'alimentation des cellules inoculées situées dans l'espace de culture cellulaire X. Le milieu frais non utilisé et récupéré à la sortie 41, par l'intermédiaire du collecteur 43, est recyclé en permanence dans une boucle sous pression alimentée à partir d'un réservoir 13 branché sur la canalisation 16 et alimente à nouveau les cellules. Le milieu usé est, pour sa part, récupéré avec les métabolites produits par les cellules au niveau des conduits de sortie 12₁ et 12₂ (figure 3) et traité ultérieurement de manière appropriée.

Simultanément, les cellules sont alimentées en oxygène par l'intermédiaire des capillaires C, répartis uniformément dans l'espace laissé libre par les tubes T, dans l'espace délimité par les tamis microniques d'extrémité, à partir d'une arrivée gazeuse Ge (figure 2). Les capillaires diffusent le gaz de l'intérieur de chaque capillaire vers les cellules mais s'opposent à la diffusion de liquide à l'intérieur desdits capillaires. La sortie Gs des gaz permet également l'évacuation du CO₂ produit par les cellules en culture.
. la sortie de gaz Gs permet d'évacuer l'oxygène non utilise et le CO₂ produit par les cellules en culture.

Un tel bioréacteur d'un volume qui peut varier de 200 cm³ à 10 l, permet de réaliser des cultures en continu sur une période de plusieurs mois.

Dans une réalisation préférée mais non limitative dudit bioréacteur :
. l'espace de culture cellulaire X a 25 mm de haut et est délimité par deux tamis microniques (M₁ et M₂, figure 1) de 250 x 400 mm.
. les tubes T d'alimentation en milieu frais, sont d'un diamètre de l'ordre de 1 cm, sont rigides et ont ces pores d'un diamètre permettant le passage de molécules de nutriments d'un poids moléculaire supérieur à 150 kDa. Ces tubes sont dans la réalisation représentés et ce de manière non limitative, en graphite compacté recouvert d'une couche sensible permettant le contrôle du diamètre des pores, ont été préalablement stérilisés et la mise sous pression du milieu frais entraîne une perte de charge, lorsque le milieu frais traverse les parois des tubes T, supérieure à 200 mbars.
. les capillaires C d'alimentation en fluides gazeux, sous la forme d'une pluralité de fibres creuses poreuses espacées régulièrement dans l'espace cellulaire X, d'un diamètre de 2,6 mm dans la réalisation représentée, sont situés de part et d'autre desdits tubes T, orientés parallèlement au plan des tubes T et perpendiculaires à ceux-ci dans ledit plan.

Après stérilisation du carter D, on inocule, par exemple et ce de manière non limitative, 2.10⁶ d'hybridomes murins VO208 qui produisent un anticorps monoclonal de type IgGl/ml de volume utile. Les conditions expérimentales sont alors les suivantes : pH de croissance 7,2 ; pH de production d'anticorps 7,05 ; concentration en O₂ dissous, environ 40 % ; température 37°C.

Le milieu frais est ensuite introduit par la tubulure d'entrée 40 vers le collecteur 42 (figure 4) et comprend par exemple du glucose (concentration résiduelle toujours supérieure à 0,8 g/l), de la glutamine (2 à 4 %), des acides aminés (1 % dans le bioréacteur), un milieu RPMI 1640 (on diminue le taux de recirculation lorsque la concentration en lactate est supérieure ou égale à 18mM) et du sérum de veau foetal à 10 % dont on diminue le taux à partir du 20ème ou du 25ème jour jusqu'à moins de 2 %. On dose quotidiennement le glucose et le lactate et régulièrement on dose la glutamine et les acides aminés et on mesure le pH. Ce milieu s'écoule dans les tubes T à une vitesse qui garantit la filtration tangentielle du milieu frais ci-dessus défini à travers les parois desdits tubes à une vitesse de l'ordre de 10 µm/s et assure une homogénéité de l'alimentation des cellules situées dans l'espace de culture cellulaire X.

De manière préférée, la vitesse d'écoulement à l'intérieur des tubes est de l'ordre de 1 dm/s et la pression est choisie de manière à obtenir le rapport recherché (par exemple 10:1) entre le débit circulant et le débit traversant la paroi du tube.

Le milieu usé est récupéré aux sorties 12₁ et 12₂ et comprend notamment dans la réalisation représentée les anticorps monoclonaux produits par lesdits hybridomes.

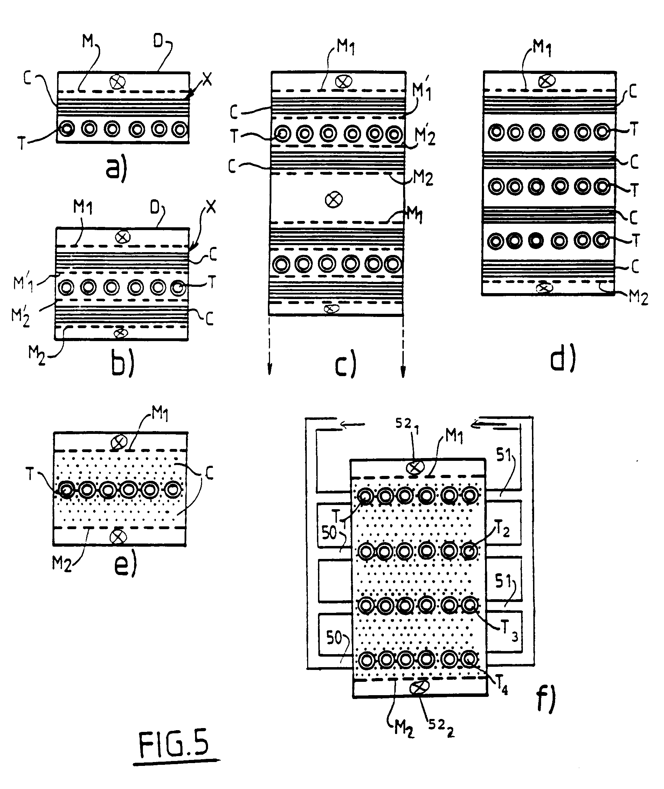

On se réfère maintenant aux modes de réalisation illustrés aux figures 5a à 5f.

La figure 5a illustre un mode de réalisation du bioréacteur conforme à l'invention, comprenant un carter D qui enferme une nappe de tubes T. Entre la nappe de tubes T et le tamis micronique M, est disposé un ensemble de capillaires, disposés perpendiculairement aux tubes T, dans le même plan et formant ainsi un "module unilatéral".

La figure 5b illustre un autre mode de réalisation du bioréacteur conforme à l'invention, pour un "module bilatéral" ou symétrique, qui comprend deux sous-ensembles de capillaires C s'étendant respectivement de chaque côté de la nappe de tubes T, deux tamis microniques d'extrémité M₁ et M₂ et deux tamis microniques M'₁ et M'₂ semblables aux tamis microniques M₁ et M₂ et séparant chaque sous-ensemble de capillaires C de la nappe de tubes T.

Les membranes M'₁ et M'₂ permettent le confinement des cellules dans l'espace où la distribution d'oxygène est homogène (près des capillaires et évitent le colmatage des tubes).

La figure 5c illustre un mode de réalisation d'un bioréacteur conforme à l'invention dans lequel deux modules symétriques sont superposés entre deux tamis d'extrémité M₁ et M₂, lequel bioréacteur comprend successivement un premier sous-ensemble de capillaires C, un tamis micronique M'₁, une nappe de tubes T, un tamis micronique M'₂, un deuxième sous-ensemble de capillaires C, un tamis micronique M₂, un espace de sortie intermédiaire puis un autre module symétrique tel que décrit ci-dessus.

La figure 5d illustre un mode de réalisation dans lequel on trouve un empilement de plusieurs modules symétriques comme visible à la figure 5c et dont l'espace de sortie intermédiaire est supprimé.

Aux figures 5a à 5d ci-dessus, les capillaires C sont perpendiculaires aux tubes T dans le plan de ceux-ci, alors qu'aux figures 5e à 5f ci-après, les tubes et les capillaires sont parallèles.

La figure 5e illustre schématiquement un module bilatéral ou symétrique qui comprend un ensemble de capillaires C emplissant tout l'espace laissé libre par la nappe de tubes T, entre les tamis microniques M₁ et M₂ d'extrémité.

La figure 5f illustre un mode de réalisation dans lequel une enceinte E est traversée par plusieurs nappes de tubes T₁, T₂, T₃, T₄ dans lesquels circule en boucle fermée le milieu nutritif frais et est emplie d'un ensemble de capillaires C parallèles auxdits tubes et comprend en outre deux tamis microniques M₁ et M₂ d'extrémité.

Comme le montre la figure 5f et comme cela est prévu pour toutes les variantes, la ou les nappes de tubes T sont alimentées en milieu frais et les capillaires C sont alimentés en gaz approprié.

## Revendications

1. Dispositif de culture cellulaire ou bioréacteur pour la production de métabolites, du type comprenant un espace de culture cellulaire (X) dans lequel les cellules sont confinées dans un milieu liquide, ledit espace étant délimité par des parois dont au moins trois sont sélectivement perméables, la première au milieu nutritionnel frais, la seconde aux fluides gazeux et la dernière au milieu nutritionnel usé et des moyens de circulation des fluides dans ledit dispositif, lequel dispositif est caractérisé en ce que :
- la première paroi est constituée par une nappe de tubes (T) à paroi perméable au milieu nutritionnel frais, rigides et de diamètre d'environ un centimètre, montés en parallèle entre un collecteur d'entrée (42) et un collecteur de sortie (43) dans un circuit en boucle fermée (10,11,16), incluant des moyens d'apport (10 ; 40) dudit milieu nutritionnel frais et des moyens de mise sous pression (14), lesquels tubes (T) sont parcourus chacun de bout en bout par un débit de milieu frais plusieurs fois supérieur à celui traversant sa paroi, pour maintenir une pression entraînant une perte de charge, lorsque le milieu frais traverse les parois des tubes (T), supérieure à 200 mbars,
- la seconde paroi est constituée par un ensemble de capillaires (C) à paroi perméable aux gaz, disposés à intervalle sensiblement égaux pour former, de l'un et/ou de l'autre côté de la nappe de tubes (T), un/plusieurs sous-ensembles à distribution homogène pour les échanges gazeux avec les cellules,
- la troisième paroi comprend au moins un tamis micronique constituant chaque paroi aval de l'espace de culture cellulaire, pour assurer le confinement des cellules et permettant la sortie du milieu usé.

2. Dispositif selon la revendication 1, caractérisé en ce que le débit du milieu parcourant chaque tube (T) correspond à une vitesse d'écoulement, d'environ 1 dm/s, qui garantit la filtration tangentielle du milieu frais à travers la paroi desdits tubes.

3. Dispositif selon la revendication 1, caractérisé en ce que lesdits sous-ensembles de capillaires peuvent être simples et constituent une seule couche.

4. Dispositif selon la revendication 1, caractérisé en ce que lesdits sous-ensembles de capillaires peuvent être eux-mêmes formés de plusieurs couches superposées de capillaires parallèles entre eux, les capillaires d'une couche régnant au droit des intervalles entre capillaires des deux couches adjacentes.

5. Dispositif selon l'une quelconque des revendications 1 à 4, caractérisé en ce que lesdits capillaires sont des fibres creuses de diamètre d'environ 1 millimètre, à paroi hydrophobe.

6. Dispositif selon l'une quelconque des revendications 1 à 5, caractérisé en ce que ledit tamis micronique (M) recouvre au moins chaque face libre aval de l'ensemble des capillaires.

7. Dispositif selon l'une quelconque des revendications 1 à 6, caractérisé en ce que ladite troisième paroi est une membrane poreuse comprenant des pores d'un diamètre compris entre 1 et 10 µm.

8. Dispositif selon l'une quelconque des revendications 1 à 7, caractérisé en ce que les capillaires (C) sont orientés parallèlement au plan de la nappe des tubes (T).

9. Dispositif selon la revendication 8, caractérisé en ce que les capillaires (C) sont parallèles aux tubes (T).

10. Dispositif selon la revendication 9, caractérisé en ce que lesdits capillaires (C) sont aussi répartis dans l'espace cellulaire libre entre les tubes (T).

11. Dispositif selon l'une quelconque des revendications 1 à 9, caractérisé en ce que la nappe de tubes (T) et l'ensemble ou les sous-ensembles de capillaires (C) sont séparés par un tamis micronique (M'₁, M'₂) semblable au tamis micronique (M) formant la troisième paroi dudit dispositif.

12. Dispositif selon l'une quelconque des revendications 1 à 11, caractérisé en ce qu'il constitue un module unilatéral, comprenant un seul ensemble de capillaires (C) situé d'un côté de la nappe de tubes (T) et au moins un tamis micronique aval (ou de sortie) (M).

13. Dispositif selon l'une quelconque des revendications 1 à 11, caractérisé en ce qu'il constitue un module bilatéral ou symétrique, comprenant au moins deux sous-ensembles de capillaires (C) s'étendant respectivement de chaque côté de la nappe de tubes (T) et au moins un tamis micronique aval (ou de sortie) (M1, M2), recouvrant chaque sous-ensemble de capillaires (C).

14. Dispositif selon la revendication 13, caractérisé en ce qu'il comprend au moins deux modules symétriques superposés, avec suppression éventuelle des espaces de sortie et tamis microniques intermédiaires.

15. Dispositif selon la revendication 13, caractérisé en ce qu'il comprend une enceinte (E) traversée par au moins deux nappes de tubes (T) et dont l'espace entre les tubes (T) est empli de capillaires (C) parallèles auxdits tubes, entre deux tamis microniques (M₁, M₂) d'extrémité.

## Claims

1. A cell culture device, or bioreactor, for the production of metabolites, of the type comprising a cell culture space (X) in which the cells are confined in a liquid medium, said space being delimited by walls of which at least three are selectively permeable, the first to a fresh nutrient medium, the second to the gaseous fluids and the last to the spent nutrient medium, and means of circulating the fluids in said device, characterized in that:
- the first wall consists of a sheet of tubes (T) whose wall is permeable to fresh nutrient medium rigid and having a diameter of the order of a centimetre, said tubes being assembled in parallel between an inlet manifold (42) and an outlet manifold (43) in a closed-loop circuit (10, 11, 16) including means (10, 40) of supplying said fresh nutrient medium, and means (14) of placing said fresh medium under pressure, said fresh medium passing through each of said tubes, from end to end, at a flow rate which is several times greater than that passing through its wall, in view to maintain a pressure leading to a pressure drop of more than 200 mbar when the fresh medium passes through the walls of the tubes (T),
- the second wall consists of a set of capillaries (C) whose wall is permeable to gases, said capillaries being arranged at approximately equal intervals so as to form, on one and/or the other side of the sheet of tubes (T), one/several subsets with homogeneous distribution for the gas exchanges with the cells,
- the third wall comprises at least one micron sieve forming each downstream wall of the cell culture space, in order to ensure that the cells are confined while allowing the spent medium to leave.

2. A device according to claim 1, characterized in that the flow rate of medium passing through each tube (T) corresponds to a flow velocity of the order of 1 dm/s, which guarantees the tangential filtration of the fresh medium through the wall of said tubes.

3. A device according to claim 1, characterized in that said subsets of capillaries can be simple and constitute a single layer.

4. A device according to claim 1, characterized in that said subsets of capillaries can themselves be formed of several superimposed layers of mutually parallel capillaries, the capillaries of one layer extending at right-angles to the intervals between capillaries of the two adjacent layers.

5. A device according to any one of claims 1 to 4, characterized in that said capillaries are hollow fibres whose diameter is of the order of a millimetre and which have a hydrophobic wall.

6. A device according to any one of claims 1 to 5, characterized in that said micron sieve (M) covers at least each downstream free face of the set of capillaries.

7. A device according to any one of claims 1 to 6, characterized in that said third wall is a porous membrane whose pores have a diameter of between 1 and 10 µm.

8. A device according to any one of claims 1 to 7, characterized in that the capillaries (C) are orientated parallel to the plane of the sheet of tubes (T).

9. A device according to claim 8, characterized in that the capillaries (C) are parallel to the tubes (T).

10. A device according to claim 9, characterized in that said capillaries (C) are also distributed in the free cell space between the tubes (T).

11. A device according to any one of claims 1 to 9, characterized in that the sheet of tubes (T) and the set or the subsets of capillaries (C) are separated by a micron sieve (M'₁, M'₂) similar to the micron sieve (M) forming the third wall of said device.

12. A device according to any one of claims 1 to 11, characterized in that it forms a one-sided module comprising a single set of capillaries (C) located on one side of the sheet of tubes (T), and at least one downstream (or outlet) micron sieve (M).

13. A device according to any one of claims 1 to 11, characterized in that it forms a two-sided or symmetrical module comprising at least two subsets of capillaries (C) running respectively along each side of the sheet of tubes (T), and at least one downstream (or outlet) micron sieve (M₁, M₂) covering each subset of capillaries (C).

14. A device according to claim 13, characterized in that it comprises at least two superimposed symmetrical modules, the outlet spaces and intermediate micron sieves being omitted if appropriate.

15. A device according to claim 13, characterized in that it comprises an enclosure (E) through which at least two sheets of tubes (T) pass and in which the space between the tubes (T) is filled with capillaries (C) parallel to said tubes, between two end micron sieves (M₁, M₂).

## Patentansprüche

1. Zellkulturanlage oder Bioreaktor für die Herstellung von Metaboliten, des Typs, der einen Zellkulturraum (X), in dem die Zellen in einem flüssigen Milieu eingeschlossen sind, wobei der Raum von Wandungen begrenzt ist, von welchen mindestens drei selektiv permeabel sind, die erste für frisches Nährmedium, die zweite für gasförmige Fluide und die letzte für gebrauchtes Nährmedium, und eine Einrichtung zur Zirkulation der Fluide in der Vorrichtung aufweist,
dadurch gekennzeichnet, daß
- die erste Wandung aus einer Schicht von steifen Röhren (T) mit einem Durchmesser von ungefähr einem Zentimeter besteht, die eine für frisches Nährmedium permeable Wandung haben und die parallel zwischen einem Einlaßsammelkörper (42) und einem Auslaßsammelkörper (43) in einem geschlossenen Kreislauf (10,11,16) mit Einrichtungen (10;40) zum Zuführen des frischen Nährmediums und einer Druckerzeugungseinrichtung (14) angebracht sind, wobei durch jede der Röhren (T) von einem Ende zum anderen eine Menge an frischem Medium hindurchfließt, die mehrere Male größer ist als diejenige, die ihre Wandung durchdringt, um einen Druck aufrecht zu erhalten, der bei Durchgang des frischen Mediums durch die Wandungen der Röhren (T) einen Ladungsverlust von mehr als 200 mbar zur Folge hat,
- die zweite Wandung aus einer Gruppe von Kapillaren (C) mit gasdurchlässiger Wandung besteht, die im wesentlichen in gleichen Abständen angeordnet sind, um an der einen und/oder der anderen Seite der Schicht von Röhren (T) eine oder mehrere homogen verteilte Teilgruppen für den Gasaustausch mit den Zellen zu bilden,
- die dritte Wandung mindestens ein Mikrosieb aufweist, das jede stromabwärts gelegene Wandung der Zellkulturanlage bildet, um den Einschluß der Zellen sicherzustellen und den Austritt des verbrauchten Mediums zu erlauben.

2. Vorrichtung nach Anspruch 1, dadurch gekennzeichnet, daß die durch jede Röhre (T) hindurchlaufende Menge an Medium einer Fließgeschwindigkeit von ungefähr 1 dm/s entspricht, die die tangentielle Filtration des frischen Mediums über die Wandung der Röhren gewährleistet.

3. Vorrichtung nach Anspruch 1, dadurch gekennzeichnet, daß die Teilgruppen von Kapillaren nicht weiter unterteilt sein können und eine einzige Schicht bilden.

4. Vorrichtung nach Anspruch 1, dadurch gekennzeichnet, daß die Teilgruppen von Kapillaren ihrerseits aus mehreren übereinanderliegenden Schichten zueinander paralleler Kapillaren gebildet sein können, wobei die Kapillaren einer Schicht in die Zwischenräume zwischen den Kapillaren der zwei benachbarten Schichten hineinwirken.

5. Vorrichtung nach einem der Ansprüche 1 bis 4, dadurch gekennzeichnet, daß die Kapillaren Hohlfasern mit hydrophober Wandung und mit einem Durchmesser von ungefähr 1 mm sind.

6. Vorrichtung nach einem der Ansprüche 1 bis 5, dadurch gekennzeichnet, daß das Mikrosieb (M) mindestens jede freie Fläche stromabwärts der Gruppe von Kapillaren bedeckt.

7. Vorrichtung nach einem der Ansprüche 1 bis 6, dadurch gekennzeichnet, daß die dritte Wandung eine poröse Membran ist, die Poren mit einem Durchmesser zwischen 1 und 10 µm aufweist.

8. Vorrichtung nach einem der Ansprüche 1 bis 7, dadurch gekennzeichnet, daß die Kapillaren (C) parallel zur Ebene der Schicht der Röhren (T) ausgerichtet sind.

9. Vorrichtung nach Anspruch 8, dadurch gekennzeichnet, daß die Kapillaren (C) parallel zu den Röhren (T) sind.

10. Vorrichtung nach Anspruch 9, dadurch gekennzeichnet, daß die Kapillaren (C) auch im freien Zellraum zwischen den Röhren (T) verteilt sind.

11. Vorrichtung nach einem der Ansprüche 1 bis 9, dadurch gekennzeichnet, daß die Schicht aus Röhren (T) und die Gruppe oder die Teilgruppen von Kapillaren (C) durch ein Mikrosieb (M'₁,M'₂) getrennt sind, das dem die dritte Wandung der Vorrichtung bildenden Mikrosieb (M) ähnlich ist.

12. Vorrichtung nach einem der Ansprüche 1 bis 11, dadurch gekennzeichnet, daß sie ein einseitiges Modul bildet, das eine an einer Seite der Schicht aus Röhren (T) gelegene, einzige Gruppe von Kapillaren (C) und mindestens ein stromabwärts gelegenes Mikrosieb oder Auslaßmikrosieb (M) aufweist.

13. Vorrichtung nach einem der Ansprüche 1 bis 11, dadurch gekennzeichnet, daß sie ein zweiseitiges oder symmetrisches Modul bildet, das mindestens zwei Teilgruppen von Kapillaren (C), die sich jeweils an jeder Seite der Schicht aus Röhren (T) erstrecken, und mindestens ein stromabwärts gelegenen Mikrosieb (oder Auslaßmikrosieb) (M1,M2) aufweist, das jede Teilgruppe von Kapillaren (C) überdeckt.

14. Vorrichtung nach Anspruch 13, dadurch gekennzeichnet, daß sie mindestens zwei übereinanderliegende symmetrische Module aufweist, wobei gegebenenfalls zwischenliegende Auslaßräume und Mikrosiebe weggelassen sind.

15. Vorrichtung nach Anspruch 13, dadurch gekennzeichnet, daß sie eine Umfassung (E) aufweist, die zwischen den zwei äußersten Mikrosieben (M1,M2) von mindestens zwei Schichten aus Röhren (T) durchquert wird und deren Raum zwischen den Röhren (T) mit zu den Röhren parallelen Kapillaren (C) aufgefüllt ist.
